# EUROPEAN PATENT APPLICATION

(11) **EP 0 834 307 A2**
(43) Date of publication of application: **08.04.1998**
(21) Application number: 97307337.2
(22) Date of filing: 19.09.1997
(51) Int. Cl.: A61K 7/50, C11D 1/00

(54) **Liquid compositions comprising edta-derived chelating surfactants**

(30) Priority: 04.10.1996 US 726283
(71) Applicant: UNILEVER PLC, London EC4P 4BQ (GB); UNILEVER N.V., 3013 AL Rotterdam (NL)
(72) Inventor: He, Mentago, Wayne, New Jersey 07470 (US); Fair, Michael Joseph, Hackensack, New Jersey 07601 (US); Massaro, Michael, Congers, New York 10920 (US)
(74) Representative: Rots, Maria Johanna Francisca

(57) **Abstract**

In one embodiment, the present invention relates to liquid detergent compositions comprising the EDTA derived anionic surfactants and other types of anionic/amphoteric surfactants. Through careful balancing of the EDTA-derived surfactants and other anionic and amphoteric surfactants, ultra mildness to skin is achieved without sacrificing consumer desired user properties, such as lather. In a second embodiment, the invention relates to a process for preparing the EDTA derived anionic surfactants into specific formulation base. By carefully controlling the surfactant concentration and temperature, the gelling of the EDTA-derived surfactants is avoided and lather benefits are not sacrificed.

## Description

### FIELD OF THE INVENTION

The present invention relates to liquid personal wash compositions (e.g., shower gels), particularly compositions comprising (1) EDTA-derived chelating anionic surfactants optionally in combination with other types of anionic surfactants (wherein preferably 30% or more of all anionics comprise the EDTA-derived anionic) and (2) one or more amphoteric surfactants. The invention further relates to the incorporation of the specific amounts (high enough to provide functionality, but not so high as to destroy processing) of EDTA-derived chelating surfactants into specific liquid skin cleansing formulation bases. Through careful balancing of the anionic, amphoteric and optionally nonionic surfactants, and through specific handling of the chelating surfactants during the processing, ultra formulation mildness to skin is achieved without sacrificing other desired user properties, such as rich and creamy lather and proper viscosity range.

### BACKGROUND

Hydrophobically modified ethylenediaminetriacetic acid (EDTA) chelating surfactants, salts thereof, and methods for making these compounds are taught for example, in U.S. Patent Nos 5,177,243, 5,191,081, 5,191,106 (all assigned to Hampshire Chemical Corporation).

U.S. Patent No. 5,250,728 to B. A. Parker et al. (assigned to Hampshire Chemical Corporation) teaches a novel route of synthesizing the hydrophobically modified ethylenediaminetriacetic acid.

U.S. Patent No. 5,284,972 to B. A. Parker et al. (assigned to Hampshire Chemical Corporation) teaches a synthetic route leading to the salts of the hydrophobically modified ethylenediaminetriacetic acid which are the chelating surfactants used in the subject invention.

Inform, Vol. 6, No.10 (October 1995, J. Crudden and B. Parker) teaches the physical and physiological properties of the chelating surfactants. Mild skin cleansers and mild shampoos are among the potential applications, as discussed in the article.

There is no teaching or suggestion, however, that the surfactants must be used in amounts defined by a specific window and formulated with specific other components (e.g., to retain user benefit properties). Further, there is no teaching or suggestion of a specific process for preparing the formulations to ensure user benefits are maintained and processing is acceptable.

Specifically, applicants have studied specific EDTA-derived surfactants and found that, although ultra-mild to skin, inclusion of these surfactants into aqueous-based personal washing cleansers in random amounts and without specific formulation guidelines is not a simple task. Further, in a second embodiment of the invention, applicants have discovered a process to ensure the EDTA-derived surfactants are formulated while retaining good latherability.

Thus, for example, while desirable to incorporate EDTA-derived surfactants for their mildness benefits, these particular anionics lather poorly. If too great an amount of anionics are delivered (since anionics generally lather well), they can swamp out the mildness affect of the EDTA-derived surfactant. If too much EDTA-derived surfactant is used, however, it can lead to gelling problems which, to be ameliorated, can cause poor latherability.

Further, in the processing embodiment of the invention, if ingredients are mixed at too high temperatures, there can be gelling problems which again, to be ameliorated, cause poor lathering.

In addition, from a formulation point of view, it is critical to avoid salts (e.g., multivalent salt) commonly used when formulating aqueous cleansers because, again, these can cause gelling and low lather problems.

Unexpectedly, applicants have discovered, in one embodiment, that careful formulation (e.g., using specific window of EDTA-derived surfactant; using other anionic and amphoteric; avoiding certain salts), the benefits of the EDTA-derived surfactant (e.g., mildness) can be maintained while formulating an aqueous surfactant solution which maintains good lather.

In a second embodiment of the invention, applicants have discovered that, by controlling process variables, these novel formulations can be made while maintaining the desired user properties.

### BRIEF SUMMARY OF THE INVENTION

Specifically, applicants invention relates to liquid personal wash compositions comprising a surfactant system comprising:
(1) 2 to 15% by wt. of the total composition of one or more salts of hydrophobically modified ethylenediaminetriacetic acid, and mixtures thereof;
   the salts of hydrophobically modified ethylenediaminetriacetic acid being the novel EDTA-derived anionic surfactants disclosed and described in US Patent Nos. 5,177,243, 5,191,081, 5,191,106, 5,250,728, and 5,284,972, all of which are hereby incorporated by reference into the subject application;
(2) 0 to 20% by wt.of the total composition of one or more anionic surfactants, other than the EDTA derived anionic described in item (1); (inclusion of said anionic surfactants is preferred because the EDTA-derived surfactants alone generally do not deliver satisfactory lather performance);
(3) 1 to 20% by wt. of the total composition of one or more amphoteric and/or zwitterionic surfactants; (inclusion of the amphoteric surfactants is crucial and required because the amphoteric and/or zwitterionic surfactants reduce the skin irritation potential of the anionic surfactants in (1) and (2), and EDTA-derived surfactants alone do not deliver satisfactory lather performance);
(4) 0 to 10% by wt. of the total composition of one or more nonionic surfactants.

Further, preferably no more than 1% wt., more preferably 0% by wt. of the total composition contains inorganic or organic salts or mixtures of such salts having multi-valent (+2 or higher) metal counterions.

Examples of such counterions include, but are not limited to, calcium (Ca²⁺), magnesium (Mg^{z+}) and aluminum (Al³⁺).

It is critical that levels of salts with such counterions be kept low because such salts tend to diminish the lather performance of the EDTA-derived surfactants.

Examples of the multi-valence salts include but are not limited to calcium chloride, magnesium chloride, aluminium chloride, magnesium sulfate, magnesium stearate, calcium laurate, etc.;

In a second embodiment of the invention, applicants have discovered that addition of 2 to 15% by wt. of the total composition of the EDTA-derived surfactants will lead to significantly enhanced mildness in compositions described above without sacrificing the surfactancy of the EDTA-derived surfactants by using the following processing route:
(A) during different stages of processing, always control the concentration of EDTA-derived surfactants at below 35% wt. of the total composition, preferably below 20% wt, because at high concentrations, the EDTA surfactants form a translucent and viscous gel, and the redissolution of the gel by dilution takes a significant period of time (i.e. a few hours) and the gel formation reduces the EDTA surfactant's latherability; and
(B) heating and mixing the aqueous cleansing composition at temperatures below 95°C, preferably below 60°C, because high temperature heating results in gelling, especially at the aqueous-air interface; it takes a significant period of time (i.e., a few hours) to have the gel redissolved in the aqueous mix by dilution and this gel formation also reduces the EDTA surfactant's latherability.

### BRIEF DESCRIPTION OF THE DRAWINGS

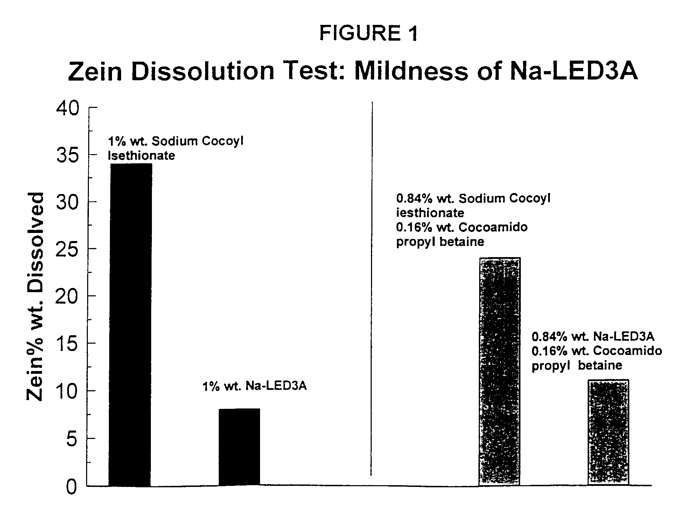

Figure 1. In-vitro skin mildness study: Zein dissolution test an EDTA-derived anionic surfactant in comparison to other commonly used mild anionic surfactants. The figure shows the value of the surfactant in improving mildness.

### DETAILED DESCRIPTION OF THE INVENTION

In one embodiment, the present invention relates to novel liquid personal washing compositions, particularly compositions in which the surfactant system comprises 2 to 15% wt. of the total composition of the salts of hydrophobically modified ethylenediaminetriacetic acid, and additionally optionally comprises one or more anionic surfactants and one or more amphoteric surfactants, wherein no more than 1% of said compositions comprise salts with multivalent counterions. Using precise formulation windows, it is possible to incorporate EDTA-derived surfactants into liquid cleaner compositions retaining the benefits of mildness and without sacrificing latherability.

In a second embodiment, the invention relates to a process for forming such compositions while retaining mildness and lathering by ensuring that concentration of surfactant and process temperature are kept within prescribed limits.

The compositions and processing are defined in greater detail below:

In the first embodiment of the invention, the liquid personal wash compositions comprise:
(1) 2 to 15% by wt. of the total composition of one of the salts of hydrophobically modified ethylenediaminetriacetic acid, or mixtures thereof;
   the hydrophobically modified ethylenediaminetriacetic acids having a general structure as follows: 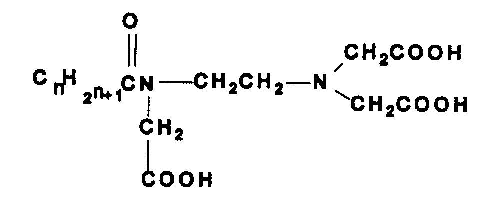 where n is from 1 to 40.

If unsaturation occurs, the hydrophobically modified group may be CₙH₂ₙ₋₁ where n is 2 to 40 and if further unsaturation occurs, the group may be CₙH₂ₙ₋₃ where n is 3 to 40 and so forth. The salts are the salts of one or more of the carboxylic acid groups. These compounds and methods of their preparation are described, for example, in U.S. Patent No, 5,284,972 to Parker et al., hereby incorporated by reference into the subject application;
(2) 0 to 20% by wt. of the total composition of one or more anionic surfactants other than the hydrophobically modified EDTA-derived compounds described above;
   inclusion of the such anionic surfactants (i.e., lathering surfactants) is preferred because the EDTA-derived surfactants alone do not deliver satisfactory lather performance;
(3) 1 to 20% by wt. of the total composition of one or more amphoteric and/or zwitterionic surfactants; inclusion of the amphoteric and zwitterionic surfactants is a criticality and required because the amphoteric surfactants reduce the skin irritation potential of the anionic surfactants in (2) and enhance the lather performance; and
(4) 0 to 10% by wt. of the total composition of one or more nonionic surfactants.

Finally the formulations of the invention preferably comprise no more than 1% wt. of the total composition of inorganic and organic salts of calcium (Ca²⁺), magnesium (Mg²⁺), aluminum (Al³⁺) and other multi-valent metal counterions, and mixtures thereof; preferably said salts are excluded from the total composition; the restriction on the concentration of said salts is important because such salts tend to diminish the lather performance of the EDTA-derived surfactants.

Examples of the multi-valence salts include, but are not limited to, calcium chloride, magnesium chloride, aluminum chloride, magnesium sulfate, magnesium stearate, calcium laurate, etc.

By using these specific formulation ingredients in specific formulation windows (e.g., 2% to 15% EDTA-derived surfactants), it is possible to make a composition comprising the mild surfactants without sacrificing lathering ability. To further insure this, the formulations should be made using the process encompassed by the second embodiment of the invention described in more detail below.

The various formulation components are described in greater detail below:

### The EDTA-derived anionic surfactants (Component (1))

The salt and/or salts of the hydrophobically modified ethylenediaminetriacetic acid (EDTA) are salts(s) of the N-acyl EDTA surfactants described in US Patent No. 5,177,243, 5,191,081, 5,191,106, 5,250,728, and 5,284,972, all of which are incorporated by reference into the subject application. The synthesis, physical and physiological properties of the EDTA-derived surfactants are also summarized in an article published recently (Inform, Vol. 6 no. 10, Oct. 1995).

The hydrophobically modified ethylenediaminetriacetic acids have general structure as follows: 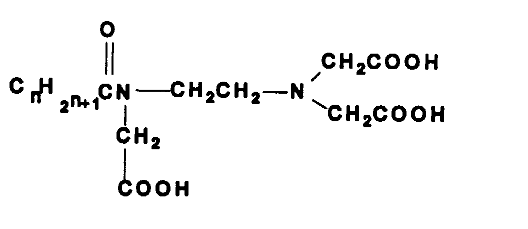 where n is from 1 to 40.

If unsaturation occurs, the hydrophobically modified group may be CₙH₂ₙ₋₁ where n is 2 to 40 and if further unsaturation occurs, the group may be CₙH₂ₙ₋₃ where n is 3 to 40 and so forth. The salts are the salts of one or more of the carboxylic acid groups. These compounds and methods of their preparation are described, for example, in U.S. Patent No, 5,284,972 to Parker et al., hereby incorporated by reference into the subject application;

For the subject invention, the pH of the aqueous cleanser compositions, including the EDTA derived surfactants, is kept at between 5.5 and 10, preferably between 6 and 8.

The counterions which may be used for the EDTA derived surfactants of the subject invention include but are not limited to sodium (Na⁺), potassium (K⁺), ammonium (NH₄⁺), monoethanolamine, diethanolamine, triethanolamine, N-Propylamine, isopropylamine, and tris(hydroxymethyl aminomethane). As noted, multivalent counterions should be avoided.

Examples of the N-acyl EDTA surfactants used by the current invention include, under the names given by B. Parker et al. (Inform, Vol. 6 no. 10, Oct. 1995), sodium lauroyl ED3A, potassium cocoyl ED3A, triethanolamine myristoyl ED3A, and sodium oleoyl ED3A.

The EDTA-derived surfactants comprise 2% to 15% of the total composition. In addition, the surfactant should comprise at least 30%, preferably $40%, more preferably $ 50% of the total anionic surfactants in the composition.

### Other anionic surfactants (Component (2))

The anionic surfactant other than EDTA-derived surfactant may be, for example, an aliphatic sulfonate, such as a primary alkane (e.g., C₈-C₂₂) sulfonate, primary alkane (e.g., C₈-C₂₂) disulfonate, C₈-C₂₂ alkene sulfonate, C₈-C₂₂ hydroxyalkane sulfonate or alkyl glyceryl ether sulfonate (AGS); or an aromatic sulfonate such as alkyl benzene sulfonate.

The anionic may also be an alkyl sulfate (e.g., C₁₂-C₁₈ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 18 carbons, preferably 12 to 18 carbons, n has an average value of greater than 1.0, preferably between 2 and 3; and M is a solubilizing cation such as sodium, potassium, ammonium or substituted ammonium. Ammonium and sodium lauryl ether sulfates are preferred.

The anionic may also be alkyl sulfosuccinates (including mono- and dialkyl, e.g., C₆-C₂₂ sulfosuccinates); alkyl and acyl taurates, alkyl and acyl sarcosinates, sulfoacetates, C₈-C₂₂ alkyl phosphates and phosphates, alkyl phosphate esters and alkoxyl alkyl phosphate esters, acyl lactates, C₈-C₂₂ monoalkyl succinates and maleates, sulphoacetates, and acyl isethionates.

Sulfosuccinates may be monoalkyl sulfosuccinates having the formula:

R⁴O₂CCH₂CH(SO₃M)CO₂M;

amido-MEA sulfosuccinates of the formula

R⁴CONHCH₂CH₂O₂CCH₂CH(SO₃M)CO₂M

wherein R⁴ ranges from C₈-C₂₂ alkyl and M is a solubilizing cation;
amido-MIPA sulfosuccinates of formula

RCONH(CH₂)CH(CH₃)(SO₃M)CO₂M

where M is as defined above.

Also included are the alkoxylated citrate sulfosuccinates; and alkoxylated sulfosuccinates such as the following: 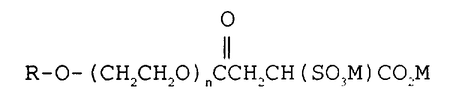 wherein n = 1 to 20; and M is as defined above. Sarcosinates are generally indicated by the formula

RCON(CH₃)CH₂CO₂M,

wherein R ranges from C₈-C₂₀ alkyl and M is a solubilizing cation.
Taurates are generally identified by formula

R²CONR³CH₂CH₂SO₃M

wherein R² ranges from C₈-C₂₀ alkyl, R³ ranges from C₁-C₄ alkyl and M is a solubilizing cation.

Another class of anionics are carboxylates such as follows:

R-(CH₂CH₂O)ₙCO₂M

wherein R is C₈ to C₂₀ alkyl; n is 0 to 20; and M is as defined above.

Another carboxylate which can be used is amido alkyl polypeptide carboxylates such as, for example, Monteine LCQ^{(R)} by Seppic.

Another surfactant which may be used are the C₈-C₁₈ acyl isethionates. These esters are prepared by reaction between alkali metal isethionate with mixed aliphatic fatty acids having from 6 to 18 carbon atoms and an iodine value of less than 20. At least 75% of the mixed fatty acids have from 12 to 18 carbon atoms and up to 25% have from 6 to 10 carbon atoms.

Acyl isethionates, when present, will generally range from about 0.5-15% by weight of the total composition. Preferably, this component is present from about 1 to about 10%.

The acyl isethionate may be an alkoxylated isethionate such as is described in Ilardi et al., U.S. Patent No. 5,393,466, hereby incorporated by reference into the subject application. This compound has the general formula: 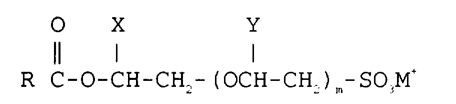 wherein R is an alkyl group having 8 to 18 carbons, m is an integer from 1 to 4, X and Y are hydrogen or an alkyl group having 1 to 4 carbons and M⁺ is a monovalent cation such as, for example, sodium, potassium or ammonium.

In general the anionic component will comprise from about 1 to 20% by weight of the composition, preferably 5 to 15%, most preferably 5 to 12% by weight of the composition.

### Zwitterionic and Amphoteric Surfactants (Component (3))

Zwitterionic surfactants are exemplified by those which can be broadly described as derivatives of aliphatic quaternary ammonium, phosphonium, and sulfonium compounds, in which the aliphatic radicals can be straight or branched chain, and wherein one of the aliphatic substituents contains from about 8 to about 18 carbon atoms and one contains an anionic group,e.g., carboxy, sulfonate,sulfate, phosphate, or phosphonate. A general formula for these compounds is: 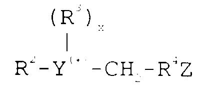 wherein R² contains an alkyl, alkenyl, or hydroxy alkyl radical of from about 8 to about 18 carbon atoms, from 0 to about 10 ethylene oxide moieties and from 0 to about 1 glyceryl moiety; Y is selected from the group consisting of nitrogen, phosphorus, and sulfur atoms; R³ is an alkyl or monohydroxyalkyl group containing about 1 to about 3 carbon atoms; X is 1 when Y is a sulfur atom, and 2 when Y is a nitrogen or phosphorus atom; R⁴ is an alkylene or hydroxyalkylene of from about 1 to about 4 carbon atoms and Z is a radical selected from the group consisting of carboxylate, sulfonate, sulfate, phosphonate, and phosphate groups.

Examples of such surfactants include:
4-[N,N-di(2-hydroxyethyl)-N-octadecylammonio]-butane-1-carboxylate;
5-[S-3-hydroxypropyl-S-hexadecylsulfonio]-3-hydroxypentane-1-sulfate;
3-[P,P-diethyl-P-3,6,9-trioxatetradexocylphosphonio]-2-hydroxypropane-1-phosphate;
3-[N,N-dipropyl-N-3-dodecoxy-2-hydroxypropylammonio]propane-1-phosphonate;
3-(N,N-dimethyl-N-hexadecylammonio)propane-1-sulfonate;
3-(N,N-dimethyl-N-hexadecylammonio)-2-hydroxypropane-1-sulfonate;
4-[N,N-di(2-hydroxyethyl)-N-(2-hydroxydodecyl)ammonio]butane-1-carboxylate;
3-[S-ethyl-S-(3-dodecoxy-2-hydroxypropyl)sulfonio]propane-1-phosphate;
3-[P,P-dimethyl-P-dodecylphosphonio]-propane-1-phosphonate; and
5-[N,N-di(3-hydroxypropyl)-N-hexadecylammonio]-2-hydroxy-pentane-1-sulfate.

Amphoteric detergents which may be used in this invention include at least one acid group. This may be a carboxylic or a sulphonic acid group. They include quaternary nitrogen and therefore are quaternary amido acids. They should generally include an alkyl or alkenyl group of 7 to 18 carbon atoms. They will usually comply with an overall structural formula: 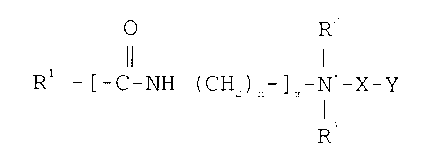
where R¹ is alkyl or alkenyl of 7 to 18 carbon atoms;
R² and R³ are each independently alkyl, hydroxyalkyl or carboxyalkyl of 1 to 3 carbon atoms;
n is 2 to 4;
m is 0 to 1;
X is alkylene of 1 to 3 carbon atoms optionally substituted with hydroxyl, and
Y is -CO₂ - or -SO₃-

Suitable amphoteric detergents within the above general formula include simple betaines of formula: 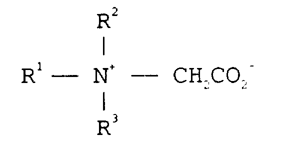 and amido betaines of formula: 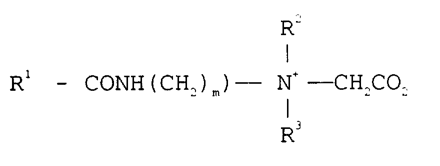 where m is 2 or 3.

In both formulae R¹, R² and R³ are as defined previously. R¹ may in particular be a mixture of C₁₂ and C₁₄ alkyl groups derived from coconut so that at least half, preferably at least three quarters of the groups R¹ have 10 to 14 carbon atoms. R² and R³ are preferably methyl.

A further possibility is that the amphoteric detergent is a sulphobetaine of formula 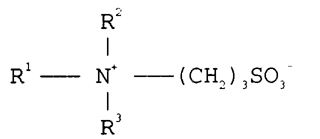 or 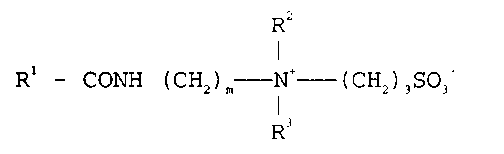 where m is 2 or 3, or variants of these in which -(CH₂)₃SO₃⁻ is replaced by

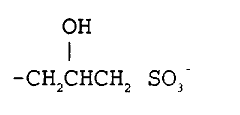

In these formulae R¹, R² and R³ are as discussed previously.

A further possibility is that the amphoteric detergent is a sulphobetaine of formula 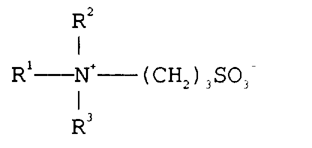 or 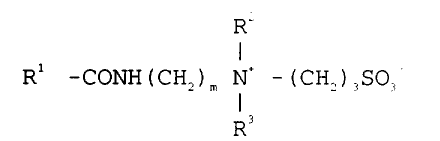 where m is 2 or 3, or variants of these in which -(CH₂)₃SO₃⁻ is replaced by

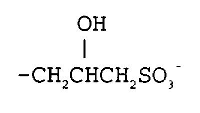

In these formulae R¹, R² and R³ are as discussed previously.

Amphoacetates and diamphoacetates are also intended to be covered in possible zwitterionic and/or amphoteric compounds which may be used.

The amphoteric/zwitterionic generally comprises 0.1 to 20% by weight, preferably 0.5% to 15%, more preferably 1.0 to 10% by wt. of the composition.

### Optional Nonionic Surfactants (Component (4))

In addition to one or more anionic and amphoteric and/or zwitterionic, the surfactant system may optionally comprise a nonionic surfactant.

The nonionic which may be used includes in particular the reaction products of compounds having a hydrophobic group and a reactive hydrogen atom, for example aliphatic alcohols, acids, amides or alkyl phenols with alkylene oxides, especially ethylene oxide either alone or with propylene oxide. Specific nonionic detergent compounds are alkyl (C₆-C₂₂) phenols-ethylene oxide condensates, the condensation products of aliphatic (C₆-C₁₈) primary or secondary linear or branched alcohols with ethylene oxide, and products made by condensation of ethylene oxide with the reaction products of propylene oxide and ethylenediamine. Other so-called nonionic detergent compounds include long chain tertiary amine oxides, long chain tertiary phosphine oxides and dialkyl sulphoxides.

The nonionic may also be a sugar amide, such as a polysaccharide amide. Specifically, the surfactant may be one of the lactobionamides described in U.S. Patent No. 5,389,279 to Au et al. which is hereby incorporated by reference or it may be one of the sugar amides described in Patent No. 5,009,814 to Kelkenberg, hereby incorporated into the subject application by reference.

Other surfactants which may be used are described in U.S. Patent No. 3,723,325 to Parran Jr. and alkyl polysaccharide nonionic surfactants as disclosed in U.S. Patent No. 4,565,647 to Llenado, both of which are also incorporated into the subject application by reference.

Preferred alkyl polysaccharides are alkylpolyglycosides of the formula

R²O(CₙH₂ₙO)ₜ(glycosyl)ₓ

wherein R² is selected from the group consisting of alkyl, alkylphenyl, hydroxyalkyl, hydroxyalkylphenyl, and mixtures thereof in which alkyl groups contain from about 10 to about 18, preferably from about 12 to about 14, carbon atoms; n is 0 to 3, preferably 2; t is from 0 to about 10, preferably 0; and x is from 1.3 to about 10, preferably from 1.3 to about 2.7. The glycosyl is preferably derived from glucose. To prepare these compounds, the alcohol or alkylpolyethoxy alcohol is formed first and then reacted with glucose, or a source of glucose, to form the glucoside (attachment at the 1-position). The additional glycosyl units can then be attached between their 1-position and the preceding glycosyl units 2-, 3-, 4- and/or 6-position, preferably predominantly the 2-position.

The nonionic surfactant can also be a water soluble polymer chemically modified with hydrophobic moiety or moieties. For example, EO-PO block copolymer, hydrophobically modified PEG such as POE(200)-glyceryl-stearate can be included in the formulations claimed by the subject invention.

Nonionic comprises 0 to 10% by wt. of the composition.

### Other Optional Ingredients

In addition, the compositions of the invention may include optional ingredients as follows:

Organic solvents, such as ethanol; auxiliary thickeners, such as carboxymethylcellulose, magnesium aluminum silicate, hydroxyethylcellulose, methylcellulose, carbopols, glucamides, or Antil^{(R)} from Rhone Poulenc; perfumes; sequestering agents, such as tetrasodium ethylenediaminetetraacetate (EDTA), EHDP or mixtures in an amount of 0.01 to 1%, preferably 0.01 to 0.05%; and coloring agents, opacifiers and pearlizers such as zinc stearate, magnesium stearate, TiO₂, EGMS (ethylene glycol monostearate) or Lytron 621 (styrene/acrylate copolymer); all of which are useful in enhancing the appearance or cosmetic properties of the product.

The compositions may further comprise antimicrobials such as 2-hydroxy-4,2'4' trichlorodiphenylether (DP300); preservatives such as dimethyloldimethylhydantoin (Glydant XL1000), parabens, sorbic acid etc.

The compositions may also comprise coconut acyl mono- or diethanol amides as suds boosters, and strongly ionizing salts such as sodium chloride and sodium sulfate may also be used to advantage.

Antioxidants such as, for example, butylated hydroxytoluene (BHT) may be used advantageously in amounts of about 0.01% or higher if appropriate.

Cationic conditioners which may be used include Quatrisoft LM-200 Polyquaternium-24, Merquat Plus 3330 - Polyquaternium 39; and Jaguar^{(R)} type conditioners.

Polyethylene glycols which may be used include:

| | | |
|---|---|---|
| Polyox | WSR-205 | PEG 14M, |
| Polyox | WSR-N-60K | PEG 45M, or |
| Polyox | WSR-N-750 | PEG 7M. |

PEG with molecular weight ranging from 300 to 10,000 Dalton, such as those marketed under the tradename of CARBOWAX SENTRY^{(R)} by Union Carbide.

Thickeners which may be used include Amerchol Polymer HM 1500 (Nonoxynyl Hydroethyl Cellulose); Glucam DOE 120 (PEG 120 Methyl Glucose Dioleate); Rewoderm^{(R)} (PEG modified glyceryl cocoate, palmate or tallowate) from Rewo Chemicals; Antil^{(R)} 141 (from Goldschmidt).

Another optional ingredient which may be added are the deflocculating polymers such as are taught in U.S. Patent No. 5,147,576 to Montague, hereby incorporated by reference.

Another ingredient which may be included are exfoliants such as polyoxyethylene beads, walnut shells and apricot seeds

The compositions may also contain 0.1 to 15% by wt., preferably 1 to 10% by wt. of a structurant. Such structurants can be used to avoid addition of external structurants (e.g., cross linked polyacylates and clays) if suspending particles is desired as well as to provide desirable consumer attributes.

The structurant is generally an unsaturated and/or branched long chain (C₈-C₂₄) liquid fatty acid or ester derivative thereof; and/or unsaturated and/or branched long chain liquid alcohol or ether derivatives thereof. It may also be a short chain saturated fatty acid such as capric acid or caprylic acid. While not wishing to be bound by theory, it is believed that the unsaturated part of the fatty acid of alcohol or the branched part of the fatty acid or alcohol acts to "disorder" the surfactant hydrophobic chains and induce formation of lamellar phase.

Examples of liquid fatty acids which may be used are oleic acid, isostearic acid, linoleic acid, linolenic acid, ricinoleic acid, elaidic acid, arichidonic acid, myristoleic acid and palmitoleic acid. Ester derivatives include propylene glycol isostearate, propylene glycol oleate, glyceryl isostearate, glyceryl oleate and polyglyceryl diisostearate.

Examples of alcohols include oleyl alcohol and isostearyl alcohol. Examples of ether derivatives include isosteareth or oleth carboxylic acid; or isosteareth or oleth alcohol.

The structuring agent may be defined as having melting point below about 25°C centigrade.

Another optional ingredient is oil/emollient which may be added as a benefit agent to the liquid compositions.

Various classes of oils are set forth below.

Vegetable oils: Arachis oil, castor oil, cocoa butter, coconut oil, corn oil, cotton seed oil, olive oil, palm kernel oil, rapeseed oil, safflower seed oil, sesame seed oil and soybean oil.

Esters: Butyl myristate, cetyl palmitate, decyloleate, glyceryl laurate, glyceryl ricinoleate, glyceryl stearate, glyceryl isostearate, hexyl laurate, isobutyl palmitate, isocetyl stearate, isopropyl isostearate, isopropyl laurate, isopropyl linoleate, isopropyl, myristate, isopropyl palmitate, isopropyl stearate, propylene glycol monolaurate, propylene glycol ricinoleate, propylene glycol stearate, and propylene glycol isostearate.

Animal Fats: Acytylated lanolin alcohols, lanolin, lard, mink oil and tallow.

Fatty acids and alcohols: Behenic acid, palmitic acid, stearic acid, behenyl alcohol, cetyl alcohol, eicosanyl alcohol and isocetyl alcohol.

Other examples of oil/emollients include mineral oil, petrolatum, silicone oil such as dimethyl polysiloxane, lauryl and myristyl lactate.

It should be understood that where the emollient may also function as a structurant, it should not be doubly included such that, for example, if the structurant is 15% oleyl alcohol, no more than 5% oleyl alcohol as "emollient" would be added since the emollient (whether functioning as emollient or structurant) should not comprise more than 20%, preferably no more than 15% of the composition.

The emollient/oil is generally used in an amount from about 1 to 20%, preferably 1 to 15% by wt. of the composition. Generally, it should comprise no more than 20% of the composition.

Preferably, the compositions of the invention should comprise no more than 1%, and should more preferably be free of inorganic or organic salts of multivalent metal counterions. Such metal counterions are defined as having valence of +2 or higher and include counterions such as calcium, magnesium and aluminum. While not wishing to be bound by theory, it is believed essential to keep the amount of such counterions low or absent so that they don't interfere with lather performance of EDTA-derived anionic surfactant.

In a second embodiment of the invention, the invention relates to a process of making the composition of the invention to ensure that EDTA-derived surfactant is incorporated, provides desired mildness characteristics and that latherability is not at the same time compromised. At least two required processing steps are required as follows:
(A) during different stages of processing, always control the concentration of EDTA-derived surfactants at below 35% wt. total composition, preferably below 20% wt, because at high concentrations, the EDTA surfactants form a translucent and viscous gel, and the redissolution of the gel by dilution takes a significant period of time (i.e. a few hours) and the gel formation reduces the EDTA surfactant's latherability.
(B) heating and mixing the aqueous cleansing composition at temperatures below 95°C, preferably below 60°C, because high temperature heating results in the gelling especially at the aqueous-air interface; it takes a significant period of time (i.e., a few hours) to have the gel redissolved in the aqueous mix by dilution; also this gel formation reduces the EDTA surfactant's latherability.

Finally, in a preferred embodiment of the invention, the invention comprises the compositions of the first embodiment of the invention made by the process of the second embodiment of the invention.

The following examples are intended to illustrate further the invention and are not intended to limit the invention in any way.

All percentages are intended to be percentages by weight unless stated otherwise.

### EXAMPLES

### Protocol of skin mildness evaluation

### Mildness Assessments:

Zein dissolution test was used to preliminarily screen the irritation potential of the formulations studied. In an 8 oz. jar, 30 mLs of an aqueous dispersion of a formulation were prepared. The dispersions sat in a 45°C bath until fully dissolved. Upon equilibration at room temperature, 1.5 gms of zein powder were added to each solution with rapid stirring for one hour. The solutions were then transferred to centrifuge tubes and centrifuged for 30 minutes at approximately 3,000 rpms. The undissolved zein was isolated, rinsed and allowed to dry in a 60°C vacuum oven to a constant weight. The percent zein solubilized, which is proportional to irritation potential, was determined gravimetrically.

### The Protocol of 4-Day Patch Test:

Patch test was used to evaluate skin mildness of aqueous dispersions containing 1% anionic active (e.g., sodium cocoyl isethionate or Na-LED3A) and different levels of the structurant/coactives. Patches (Hilltop^{(R)} Chambers, 25 mm in size) were applied to the outer upper arms of the panelists under bandage type dressings (Scanpor^{(R)} tape). After each designated contact periods (24 hrs. for the first patch application, 18 hrs. for the second, third and fourth applications), the patches were removed and the sites were visually ranked in order of severity (erythema and dryness) by trained examiners under consistent lighting.

### The Lather Volume Measurement:

The lather performance was studied by a cylinder shaking test. Forty grams of a test solution was put in a 250ml PYREX cylinder with cap. Foam was generated by shaking the cylinder for 0.5 minute. After the foam settled for 2.5 minutes, the foam height was measured.

### Preparation of the EDTA Derived Surfactants:

Sodium laurel EDTA (named as Na-LED3A) was obtained through neutralizing N-laurel-N, N'N'-ethylenediaminetriacetic (Hampshire, under the tradename of LED3A) using 50% sodium hydroxide (NaOH) solution. As an example, 50g LED3A was first dispersed and mixed in 450g 25°C distilled water. Under the room temperature, 19.3g NaOH solution (50%) was slowly added into the LED3A-water mixture. After adequate mixing, the LED3A was neutralized to pH 7.3, and molar ratio between LED3A and sodium hydroxide is approximately at 1:2. Through this approach, a 10.7% wt. Na-LED3A solution was prepared. Through dilution, a calculated amount of Na-LED3A can be added to the rest of the compositions of a skin cleanser. To avoid the gelling problem, the concentration of the Na-LED3A in water is always controlled at below 35% wt., preferably below 20% wt. For the same reason, heating was at best not used or controlled at below 60C in the neutralization process.

### Formulation Processing:

Formulations shown in the examples of this invention were prepared in 400 mL beakers under room temperature (around 25°C) or in a 30-60°C oil bath. The low temperature heating was necessary to insure that the EDTA-derived surfactants not form a viscous gel, which may take a significant period of time to dissolve by dilution. During the mixing, the concentration of the EDTA-derived surfactants were always controlled at below 35% wt., preferably below 20% wt. total composition. Mixing was accomplished with a variable speed overhead motor at temperatures below 60°C. Batch size was varied from 100-250 gms. All chemicals used except the EDTA derived surfactants were commercial materials and used as supplied. Those chemicals were dispersed in Milli-Q water, which accounted for 50-80% of the whole formulation. After the batch was homogeneously mixed, it was allowed to be cooled under room temperature.

### Example 1: An in-vitro test of the skin mildness of Na-LED3A

The skin irritation potential of Na-LED3A was investigated by the zein dissolution test. As shown in Figure 1, Na-LED3A dissolved significantly less amount of zein than commonly used anionic surfactants, such as sodium cocoyl isethionate. In the presence of amphoteric surfactants, such as cocoamidopropyl betaine, Na-LED3A also dissolved significantly less amount of zein protein than a mild surfactant system such as sodium cocoyl isethionate/betaine. The results indicate that the sodium lauroyl EDTA is an ultra-mild anionic surfactant to skin.

### Example 2: Lather of Na-LED3A

The lather performance of the Na-LED3A aqueous solution is not as high as commonly used anionic surfactants such as sodium laurylether (3EO) sulfate. As shown in Table 1, the lather volume of the 2.5% Na-LED3A is significantly less than that of SLES. However, by adding relatively low levels of SLES and Cocoamidopropyl betaine as coactives to the Na-LED3A solution, the lather performance was greatly improved. This example demonstrates the effect of inclusion of anionic and amphoteric surfactants into an EDTA-derived surfactant based skin cleanser.

**Table 1.**

| Lather volume of surfactant aqueous solutions. | |
|---|---|
| **Compositions** | **Lather Volume (ml)** |
| 2.5% wt. Na-LED3A* | 124 |
| 2.5% wt. Sodium Laurylether (3EO) Sulfate | 170 |
| 2.5% wt. Na-LED3A, 1.0% wt. Sodium Laurylether (3EO) Sulfate, 0.5% wt. Cocoamidopropyl betaine. | 211 |

| | |
|---|---|
| * An EDTA-derived anionic surfactant as defined in preparation example. | |

### Example 3: The Defoaming Effect of Multi-valence Salts to the EDTA-derived Surfactants

Organic and inorganic salts containing multi-valence cationic counterions, such as aluminium chloride, magnesium chloride, calcium chloride, calcium stearate, magnesium laurate, etc. are often used in personal washing products. However, these multi-valence salts can interact with the said EDTA-derived surfactants and cause defoaming if the salt concentration is above 1% wt. total composition. As shown in Table 2, 2.5% salts significantly defoamed the EDTA-derived surfactant. Therefore, preferably, these multi-valence salts are excluded from the skin cleansing compositions claimed by this invention.

**Table 2.**

| Lather Volume of the surfactant solutions w/ and w/o the multi-valence salts. | |
|---|---|
| **Compositions** | **Lather Volume (ml)** |
| 2.5% wt. Na-LED3A* | 124 |
| 2.5% wt. Na-LED3A 2.5% wt. Aluminum Chloride | 39 |
| 2.5% wt. Na-LED3A 2.5% wt. Magnesium Chloride | 2 |

| | |
|---|---|
| * An EDTA-derived anionic surfactant as defined in preparation example. | |

### Example 4: Skin Cleansing Composition

All amounts are given in percentage of weight. Formulations (A), (B) and (C) used sodium cocoyl isethionate and Na-LED3A as the major anionic detergent with amphoteric cocoamidopropyl betaine as a coactive. The formulations provided rich, creamy, and slippery lather that was rinsed off easily. This, it was able to obtain mildness benefits and lathering.

**Table 3**

| Formulation | (A) | (B) | (C) |
|---|---|---|---|
| Sodium cocoyl isethionate (From DEFI*) | 0 | 0 | 5.0% |
| Sodium cocoyl isethionate (From IGEPON AC-78) | 5.0% | 3.0% | 0.0 |
| Cocoamidopropyl betaine | 5.0 | 4.5 | 3.8 |
| Sodium lauryl ether sulphate, 3EO | 0.0 | 1.8 | 4.8 |
| Glycerin | 0.0 | 1.4 | 1.0 |
| Palmitic-stearate acid (From IGEPON or DEFI) | 1.0 | 0.6 | 1.6 |
| Na-LED3A** | 7.0 | 10.0 | 5.0 |
| Sunflower seed oil | 0.0 | 4.5 | 0.0 |
| Propylene glycol | 0.0 | 4.8 | 0.0 |
| Ethylene glycol | 2.0 | 1.8 | 1.4 |
| Ammonium chloride | 0.0 | 2.0 | 3.0 |
| Sodium isethionate | 0.4 | 0.4 | 0.2 |
| Water | balance to 100% | balance to 100% | balance to 100% |

| | | | |
|---|---|---|---|
| *DEFI: directly esterified fatty acid isethionate, which is a mixture containing about 74% by weight of sodium acyl isethionate, 23% stearic-palmitic acid and small amounts of other materials, manufactured by Lever Brothers Co, U.S. | | | |
| **Na-LED3A: an EDTA-derived surfactant defined by preparation example. | | | |

### Example 5: Skin Cleansing Composition

All amounts are given in percentage of weight. Formulations (C), (D) and (E) used sodium lauryl sulphate (3EO) and Na-LED3A as the major anionic detergent with amphoteric cocoamidopropyl betaine as a coactive. These clear, free flow liquids provided rich, creamy and slippery lather and smooth skin feel.

**Table 4**

| Formulation | (C) | (D) | (E) |
|---|---|---|---|
| SLES (3EO) | 5.0 | 10.0 | 5.0 |
| Na-LED3A | 5.0 | 5.0 | 10.0 |
| Cocoamidopropyl betaine | 10.0 | 5.0 | 5.0 |
| Propylene glycol | 2.0 | 1.0 | 2.0 |
| Pluronic F88 | 5.0 | 10.0 | 10.0 |
| Water | Balance to 100% | Balance to 100% | Balance to 100% |

## Claims

1. A liquid detergent composition comprising:
(a) 2 to 15% by wt. of the total composition of a salt or salts of hydrophobically modified ethlylenediaminetriacetic acid having the structure 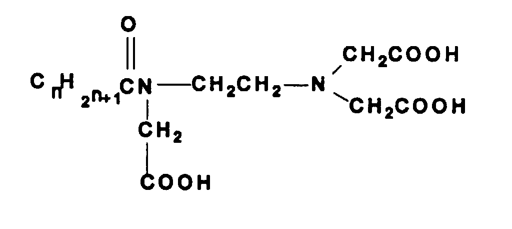 where n is 1 to 40;
(b) 0 to 20% by wt. of the total composition of anionic surfactant or surfactant other than the EDTA derived anionic surfactants defined in (a);
(c) 1 to 20% by wt. of the total composition of one or more amphoteric and/or zwitterionic surfactants;
(d) 0 to 10% by weight nonionic;
wherein component (a) comprises at least 30% of total of (a) plus (b).

2. A composition according to claim 1, comprising no more than 1% by wt. of the total composition of a salt or salts having a counterion with valence of 2⁺ or greater.

3. A composition according to claim 1 or 2, wherein component (a) comprises 50% by wt. or greater of the total anionic surfactant system;

4. A composition according to claim 2 or 3 wherein the pH of the total composition is between 5.5 and 10;

5. A composition according to claim 4, wherein the pH of the total composition is between 6 and 8;

6. A composition according to any preceding claim, wherein 1 to 10% by wt. total composition one or more nonionic surfactants are included as coactives;

7. A composition according to any preceding claim, wherein 1 to 15% by wt. of the total composition of one or more anionic surfactants other than the salt or the salts of hydrophobically modified ethlylenediaminetriacid (1(a)), are included as lather boosters;

8. A composition according to claim 2, wherein the said multi-valent salts are excluded from the total composition;

9. A process for preparing aqueous or skin cleansing formulation comprising skin cleansing formulation and an EDTA-derived surfactant which process comprises:
(a) during different stages of processing, always controlling the concentration of the EDTA-derived surfactants at below 35% wt. total composition; and
(b) mixing the components of said aqueous skin cleansing formulation at temperatures below 95°C;

10. A process according to claim 9, wherein the concentration of the said EDTA-derived anionic surfactants is below 20% wt. total composition;

11. A method of processing according to claim 9, wherein the mixing temperature is below 60°C;

12. A composition according to claim 1, made by the process of claim 9.
